# EUROPEAN PATENT APPLICATION

(11) **EP 2 628 721 A1**
(43) Date of publication of application: **21.08.2013**
(21) Application number: 12156137.7
(22) Date of filing: 20.02.2012
(51) Int. Cl.: C07C 67/327, C07C 45/68, C07C 51/08, C07C 51/083, C07C 51/41, C07C 67/08, C07C 67/31, C07C 69/732, C07C 69/74, C07C 253/14, C07C 253/30, C07C 255/16, C07C 255/30, C07C 255/46, C07C 211/40, C07D 487/04

(54) **Synthesis of 2-(3,4-difluorophenyl)cyclopropanecarboxylic acid**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to the field of organic synthesis and describes the synthesis of specific intermediates suitable for the preparation of triazolopyrimidine compounds such as ticagrelor.

## Description

The present invention relates to the field of organic synthesis, in particular to the synthesis of specific intermediates suitable for the synthesis of triazolopyrimidine compounds.

An important triazolopyrimidine compound is ticagrelor (TCG; Brilinta^{®}; 3-[7-[[(1*R*,2*S*)-2-(3,4-difluorophenyl)cyclopropyl]amino]-5-(propylthio)-3*H*-1,2,3-triazolo[4,5-d]pyrimidin-3-yl]-5-(2-hydroxyethoxy)-(1*S*,2*S*,3*R*,5*S*)-1,2-cyclopentanediol) having the following structural formula.

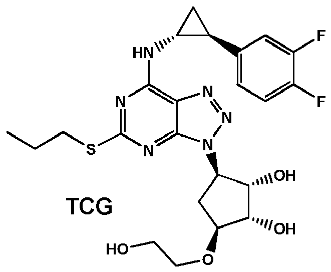

Ticagrelor shows pharmaceutical activity by functioning as a P2Y12 receptor antagonist and thus is indicated for the treatment or prevention of thrombotic events, for example stroke, heart attack, acute coronary syndrome or myocardial infection with ST elevation, other coronary artery diseases and arterial thrombosis as well as other disorders related to platelet aggregation (WO 00/34283).

The synthesis of ticagrelor (**TCG**) is demanding. There are five to six known synthetic variants, which are described in the basic patent application WO 00/34283, an improved one in patent application WO 01/92263, and a further improved one in patent application WO 10/030224 respectively derived from the originator AstraZeneca, while two are published in a "deutero" patent application WO 11/017108 of Auspex Pharmaceuticals. Further, there is one synthetic path published in a scientific journal (Bioorg. Med. Chem. Lett. 2007, 17, 6013-6018).

The ticagrelor molecule consists of three main parts, an essential part being the cyclopropyl moiety. All synthetic approaches mentioned above utilize the intermediate **CPA** (*trans*-(1*R*,2*S*)-2-(3,4-difluorophenyl)cyclopropylamine) of formula **VIII** as one of the key intermediates.

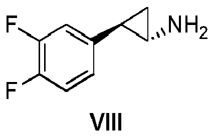

There are several synthetic paths for preparation of intermediate CPA known in the literature.

According to WO 01/92200 and WO 01/92263 (Scheme 7) CPA is prepared as shown in Scheme 7. 3,4-Difluorobenzaldehyde is reacted with malonic acid in the presence of pyridine and piperidine to yield (E)-3-(3,4-difluorophenyl)-2-propenoic acid, which is converted to (E)-3-(3,4-difluorophenyl)-2-propenoyl chloride by using thionyl chloride in the presence of toluene and pyridine. A solution of L-menthol in toluene is added to the obtained compound in the presence of pyridine to yield (1R,2S,5R)-2-isopropyl-5-methylcyclohexyl (*E*)-3-(3,4-difluorophenyl)-2-propenoate, which is with dimethylsulfoxonium methylide, sodium iodide and NaOH in DMSO converted to (1R,2S,5R)-2-isopropyl-5-methylcyclohexyl *trans*-2-(3,4-difluorophenyl)cyclopropanecarboxylate. The latter is then hydrolysed to *trans*-(1*R*,2*R*)-2-(3,4-difluorophenyl)cyclopropanecarboxylic acid, which is subsequently converted to *trans*-(1*R*,2*R*)-2-(3,4-difluorophenyl)cyclopropanecarbonyl chloride with thionyl chloride. In the last two steps, the obtained carbonyl chloride is first converted to the corresponding azide by addition of sodium azide and tertbutylammonium bromide, which is finally converted to **CPA.**

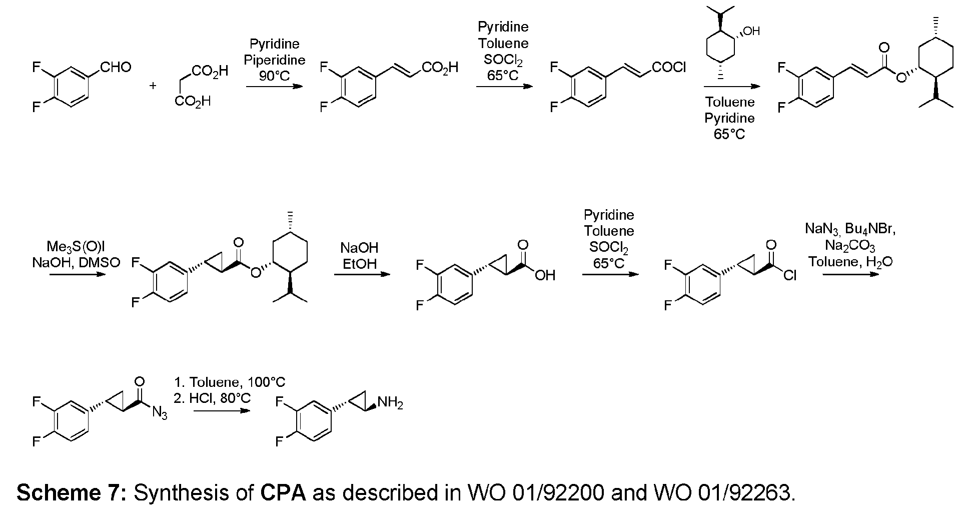

The eight steps synthesis described Scheme 7 is very long, uses toxic compounds like sodium azide and pyridine, and is not economical as expensive reagents like trimethylsulfoxonium iodide are used. Moreover, the overall yield of the reaction is low.

Another process for the preparation of **CPA** is described in Bioorg. Med. Chem. Lett. 2007, 17, 6013-6018. The synthesis of the *trans*-(1*R*,2*S*)-phenylcyclopropylamine begins with derivatisation of substituted cinnamic acid **A** with Oppolzer's sultam to give **B**. Diastereoselective cyclopropanation then provides, after recrystallisation, cyclopropylamide **C** in high chiral purity which is readily saponified to acid **D**. A four-step Curtius rearrangement gives **CPA**.

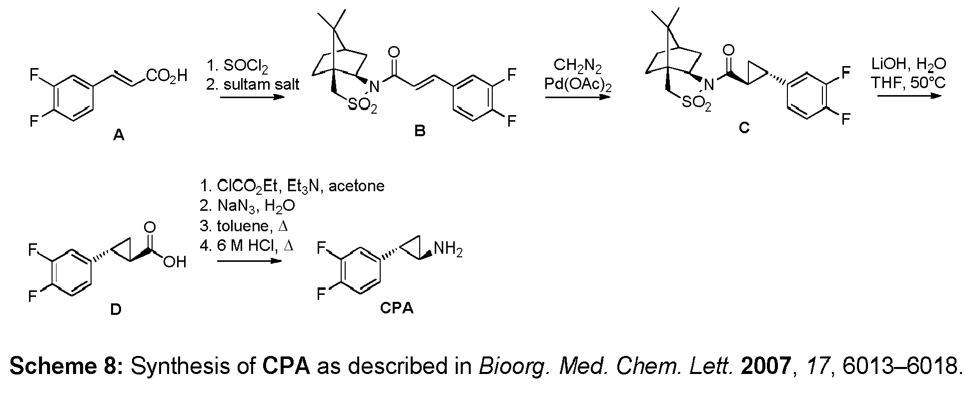

This 8 steps long synthesis presented in Scheme 8 involves the use of hazardous and explosive materials like sodium hydride, diazomethane and sodium azide. Moreover, highly expensive chiral sultamam and palladium acetate are used. In addition, this lengthy process involves column chromatographic purifications, which are generally undesirable in view of large scale industrial applicability

According to WO 08/018822 and WO 08/018823, **CPA** is prepared as shown in Scheme 9. First step involves reacting 1,2-difluorobenzene with chloroacetyl chloride in the presence of aluminium trichloride to produce 2-chloro-1-(3,4-difluorophenyl)ethanone. The keto group of the latter is then reduced by the use of chiral oxazaborolidine catalyst and borane dimethylsulfide complex to yield 2-chloro-1-(3,4-difluorophenyl)ethanol, which is then reacted with triethylphosphoacetate in the presence of sodium hydride in toluene to produce *trans*-(1*R*,2*R*)-2-(3,4-difluorophenyl)cyclopropyl carboxylate. In the final two steps the ester compound is first converted to amide by methyl formate in the presence of ammonia, said amide is then reacted with sodium hydroxide and sodium hypochlorite to produce **CPA.**

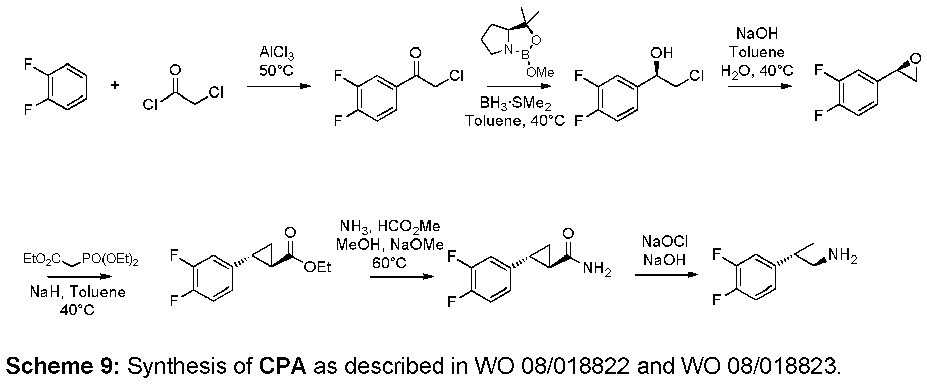

The disadvantages of the process described in WO 08/018822 and WO 08/018823 are the use of expensive chiral oxazaborolidine catalyst and toxic borane dimethylsulfide complex as well as use of explosive materials like sodium hydride.

According to WO 11/017108, **CPA** is prepared by a process as described in Scheme 10. First, (*E*)-3-(3,4-difluorophenyl)acrylic acid is reacted with oxalyl dichloride in the presence of *N*,*N-*dimethylformamide and dichlorometane to produce the corresponding acryloyl chloride, which is then reacted with a mixture of (2*R*)-bornane-10,2-sultam and triethylamine to yield [3a*S-*[1(*E*),3a,6,7a]]-1-[3-(3,4-difluorophenyl)-1-oxo-2-propenyl]-hexahydro-8,8-dimethyl-3*H*-3a,6-methano-2,1-benzisothiazole-2,2-dioxide. Said intermediate is then reacted with methyl-1-nitrosourea in NaOH and ethyl ether, and with palladium acetate reagent to convert the propenyl group into cyclopropyl group. In the final two steps the obtained intermediate is first converted to (1*R*,2*R*)-*trans*-2-(3,4-difluorophenyl)cyclopropyl carboxylic acid by lithium hydroxide in the presence of tetrahydrofuran, said acid is then reacted with diphenylphosphoryl azide and triethylamine in toluene and HCl to produce **CPA.**

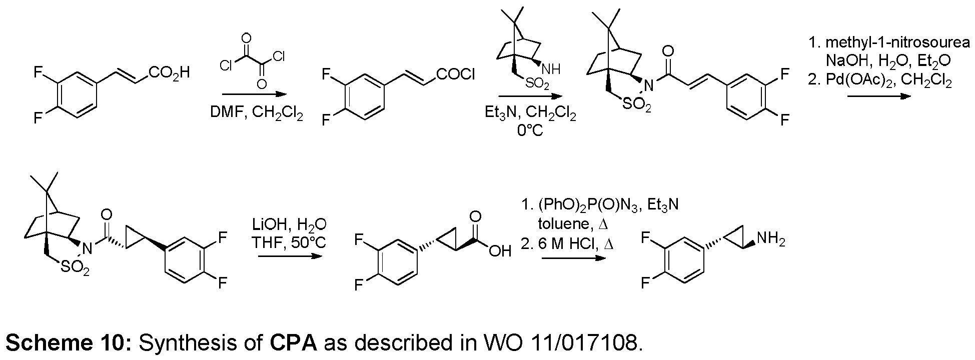

The disadvantage of the process described in WO 11/017108 is the use of expensive chiral sultam and palladium acetate reagent.

Another synthetic route for preparing **CPA** is described in WO 11/132083 (Scheme 11). 1,2-Difluoro benzene is reacted with 3-chloropropionyl chloride to produce 3-chloro-1-(3',4'-difluorophenyl)-propan-1-one, which is by addition of *N*,*N*-dimethylformamide, phloroglucinol and sodium iodide in the next step converted to 1-(3',4'-difluorophenyl)-3-nitro-propan-1-one. The keto group of the latter intermediate is in the subsequent step stereochemically reduced to hydroxyl group by the use of chiral oxazaborolidine together with borane dimethyl sulfide or borane-N,N,diethyl aniline complex in the presence of tetrahydrofurane. The obtained (1*R*)-1-(3',4'-difluorophenyl)-3-nitro-propan-1-ol is then added to a mixture of triphenylphosphine and diethylazodicarboxylate in benzene to yield *trans*-(1*S*,2*R*)-2-(3',4'-difluorophenyl)-1-nitrocyclopropane, which is in the last step converted to **CPA** by reduction of the nitro group by catalytic hydrogenaiton with palladium catalyst and zink dust.

The disadvantage of the process described in WO 11/132083 is the use of expensive chiral oxazaborolidine and palladium catalyst, sodium iodide, toxic borane dimethylsulfide complex, heavy metals and hazardous diethyl azodicarboxylate.

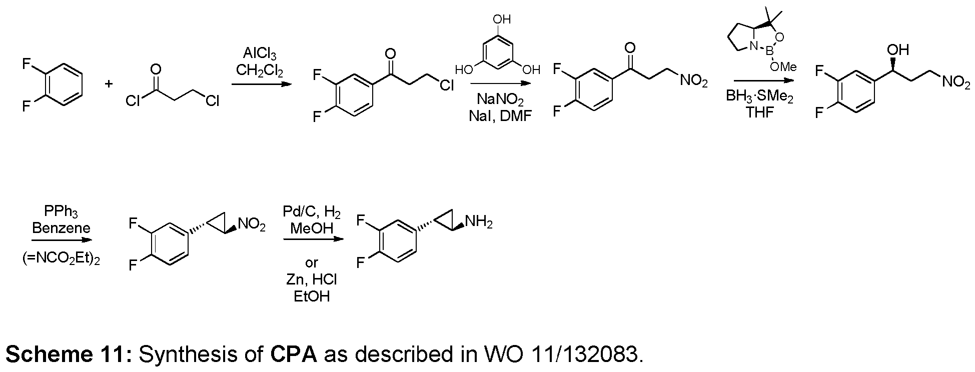

WO 12/001531 describes another alternative synthetic path for preparation of **CPA** (Scheme 12). In this case the starting reagent 3,4-difluorobenzaldehide is reacted with a mixture of methyltriphenylphosphonium bromide, 1,8-diazabicyclo[5.4.0]undec-7-ene (DBU) and toluene to yield 3,4-difluorostyrene. The obtained intermediate is then added to dichloro(p-cymene)ruthenium(II) dimer and (*S*,*S*)-2,6-bis(4-isopropyl-2-oxazolin-2-yl)pyridine, which is followed by addition of ethyl diazoacetate to yield ethyl *trans*-(1*R*,2*R*)-2-(3,4-difluorophenyl)-1-cyclopropane-carboxylate, which is converted to *trans*-(1*R*,2*R*)-2-(3,4-difluorophenyl)-1-cyclopropane-carboxylic acid by hydrolysis in the presence of sodium hydroxide and methanol. The obtained carboxylic acid is with aqueous hydroxylamine solution further converted to *trans-*(1*R*,2*R*)-2-(3,4-difluorophenyl)-1-cyclopropanecarboxamide, which is mixed with pyridine and acetic anhydride to yield *trans*-(1*R*,2*R*)-N-(acetyloxy)-2-(3,4-difluorophenyl)-1-cyclopropane carboxamide. To the obtained intermediate in the presence of tetrahydrofurane the 1,8-diazabicyclo[5.4.0]undec-7-ene (DBU) is added, which is followed by addition of isopropyl acetate and ammonium chloride to finally yield **CPA**.

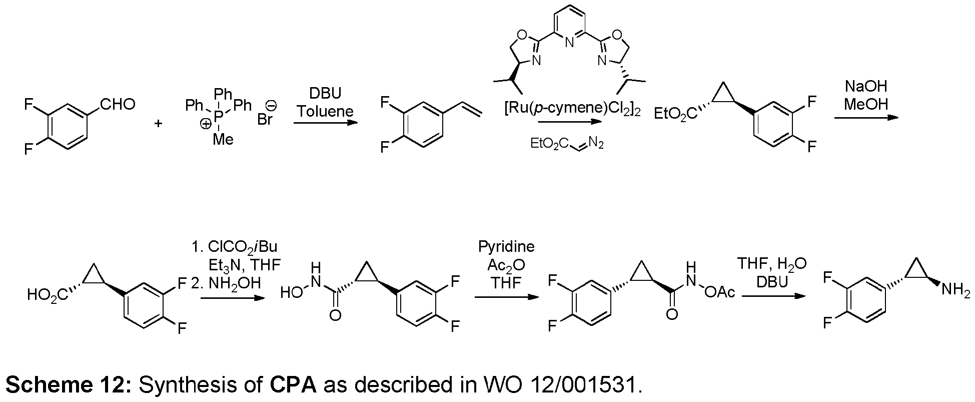

The disadvantage of the process described in WO 12/001531 is the use of expensive chiral ligand, dichloro(p-cymene)ruthenium(II) dimer, and use of toxic pyridine.

A major drawback of the hitherto known synthesis schemes for the preparation of **CPA** is that the synthesis is long and/or expensive or environmentally unfriendly reagents are used, which makes the prior art processes unsuitable for large scale preparation of **CPA** of formula **VIII**. A need therefore remains for an alternative but commercially viable process for preparation of **CPA**.

### Summary of the Invention

The object of the present invention was to provide an industrially applicable and economical process for obtaining 2-(3,4-difluorophenyl)cyclopropanecarboxylic acid (**VII**), an important intermediate in preparation of **CPA** (**VIII**).

The present invention provides a process for the preparation of a compound of formula **VI** 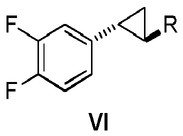 wherein R is a group convertible to carboxyl group selected from the group consisting of CN, trihalomethyl and COOR₁, wherein R₁ is linear or branched C₁-C₆ alkyl, the process comprising the steps of:
(i) providing a compound of formula **V** 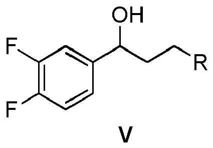 wherein R is a group convertible to carboxyl group selected from the group consisting of CN, trihalomethyl and COOR₁, wherein R₁ is linear or branched C₁-C₆ alkyl,
(ii) converting the hydroxyl group of the compound of formula **V** to a better leaving group, and
(iii) converting the obtained compound by cyclization in a presence of a base to provide a compound of formula **VI**.

The process defined above allows for preparation or synthesis of the compound of formula **VII** with an industrially applicable and economical process while using environmentally friendly reagents. Preferred embodiments will be described below. The present invention further provides novel compounds that are useful as intermediates in the preparation of ticagrelor (**TCG**).

### Description of the Invention and of Preferred Embodiments

Aspects and preferred embodiments of the present invention will be described in further detail below, noting however that such aspects as well as embodiments and examples are presented for illustrative purposes only and shall not limit the invention in any way.

According to a preferred embodiment, the compound of formula **V** is prepared by comprising the steps of
(i) providing a compound of formula **IV** 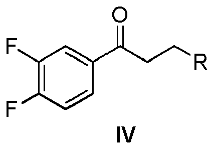 wherein R is a group convertible to carboxyl group selected from the group consisting of CN, trihalomethyl and COOR₁, wherein R₁ is linear or branched C₁-C₆ alkyl, and
(ii) reducing the keto group of the compound of formula **IV** to yield the compound of formula **V**.

The group convertible to carboxyl group can be any group that allows the formation of carbanion on a carbon atom attached to it and can be further transformed to carboxyl group. Said group is preferably selected from the group consisting of carboxylic ester (COOR₁), cyano, trihalomethyl, ethynyl group and a carboxamide group.

The R₁ group of the carboxylic ester COOR₁ can be any substituted or unsubstituted linear or branched alkyl, for example linear or branched C₁-C₆ alkyl, cyclic alkyl, for example L-menthyl, or substituted or unsubstituted aryl. Preferably R₁ is selected from the group consisting of methyl (Me), ethyl (Et), propyl (Pr), secondary such as *iso*-propyl (iPr) or tertiary alkyl such as *tert*-butyl (tBu).

Reduction of keto group of the compound of formula **IV** can be performed by any suitable reducing agent, for example metal borohydride, or alternatively by using heterogeneous or homogeneous catalysts usually employed for reduction of ketones and hydrogen or hydrogen donor.

In another embodiment of the present invention, the compound of formula **IV** is prepared from a compound of formula **I** with a process comprising:
(i) providing a compound of formula **I** 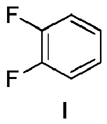
(ii) reacting the compound of formula I with a compound of formula **IIa** or **IIb** 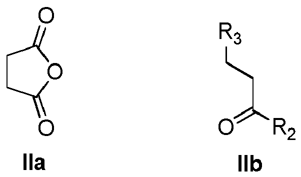 wherein R₂ and R₃ are independently selected from a group consisting of hydroxyl, Cl, Br and I,
   to yield a compound of formula **IIIa** or **IIIb**, respectively 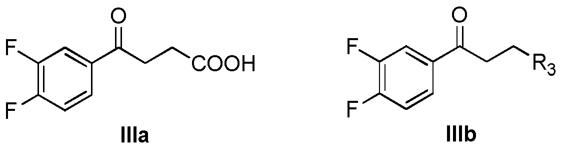 wherein R₃ is as defined above, and
(iii) converting the compound of formula **IIIa** or **IIIb** to the compound of formula **IV**.

The reaction of the compound of formula **I** with the compound of formula **IIa** or **IIb** is performed in the presence of a Lewis acid, for example AlCl₃. This Friedel-Crafts reaction can be conducted in any solvent, preferred solvents are dichloromethane and 1,2-difluorobenzene. The substituents R₂ and R₃ in the compound of formula **IIb** is a leaving group, preferably selected from a group consisting of hydroxyl, Cl, Br and I, most preferably Cl.

Alternatively, the compound of formula **IV** can also be directly obtained by reacting a compound of formula **I** with the compound of formula **IIIc** 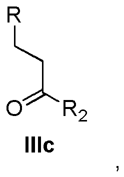 wherein R is a group convertible to carboxyl group selected from the group consisting of CN, trihalomethyl and COOR₁, wherein R₁ is linear or branched C₁-C₆ alkyl, and R₂ is selected from a group consisting of hydroxyl, Cl, Br and I.

In another preferred embodiment, the compound of formula **VI** is further converted to the compound of formula **VII** or a salt thereof by means of basic or acidic hydrolysis, optionally in the presence of a cosolvent.

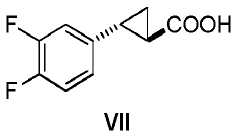

A suitable salt of intermediate **VII** is a metal salt, for example an alkali metal or earth alkali metal salt. The preferred salt of intermediate **VII** is lithium salt.

In another embodiment, the compound of formula **VII** or a salt thereof, prepared by the process of the present invention, is further converted to **CPA**, a compound of formula **VIII**, by a process known in the art. A process described in Bioorg. Med. Chem. Lett. 2007, 17, 6013-6018 (Scheme 8) or the one described in WO 11/017108 (Scheme 10) can be followed.

In a particularly preferred embodiment, the compound of formula **VII** is synthesized as shown in Scheme 13. 1,2-Difluorobenzene (**I**) and succinic anhydride (**IIa**) as starting materials are reacting in the presence of a Lewis acid such as AlCl₃ to give **IIIa** as white solid. This Friedel-Crafts reaction can be conducted in any solvent, preferably in CH₂Cl₂ or 1,2-difluorobenzene. **IIIa** is then esterified in the presence of an appropriate solvent and acid, preferably H₂SO₄, to afford esters **IVa** or **IVc** as white solid. In the next step, the keto group of the respective ester is reduced using reducing agent such as metal borohydride or other reducing agent. The reduction can be carried out either in stereoselective way or not. It can be reduced in the presence of heterogeneous or homogeneous catalyst usually employed for reduction of ketones and hydrogen or hydrogen donor to form **Va** or **Vc** in enantiomerically pure form or as a mixture of enantiomers. The hydrogen donor can be formic acid and its derivatives, borane and its derivatives, cyclohexene and its derivatives or other hydrogen donor used in reduction of ketones. Reduction can also be carried out following biochemical principles. **Va** and **Vc** are then cyclized to cyclopropane compounds **VIa** or **VIc**. First, the hydroxyl group in converted to a better leaving group by either formation of substituted or unsubstituted arylsulfonate or substituted or unsubstituted alkylsulfonate ester, and then cyclization is performed by addition of a base, preferably potassium tert-butoxide or sodium hydride. In the last step a simple basic or acidic hydrolysis is performed to afford the compound of formula **VII**.

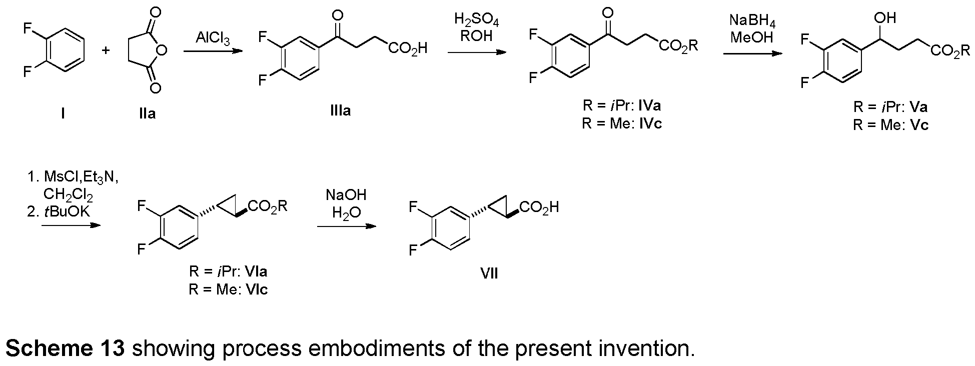

This synthetic route of Scheme 13 was found particularly advantageous for its simplicity and possibility of preparation of the compound of formula **VII** enriched in one enantiomer.

In another particularly preferred embodiment, the compound of formula **VII** is synthesized as shown in Scheme 14. 3-Chloro-1-(3,4-difluorophenyl)propan-1-one (**IIIb'**) is prepared by reacting 1,2-difluorobenzene (I) with 3-chloropropionyl chloride (**IIb'**) in the presence of a Lewis acid such as AlCl₃, optionally without the use of additional solvent. Next, a solution of 3-chloro-1-(3,4-difluorophenyl)propan-1-one (**IIIb'**) is reacted with a base, followed by cyanide such as sodium cyanide to form **IVb**. Base can also be cyanide itself. Solvent can be either protic or aprotic, preferably the solvent is selected from C₁-C₆ alcohols, most preferably solvent is ethanol. In the next step, **IVb** is reduced with reducing agent such as metal borohydride or other reducing agent or it is reduced in the presence of heterogeneous or homogeneous catalyst usually employed for reduction of ketones and hydrogen or hydrogen donor to form **Vb** in enantiomerically pure form or as mixture of enantiomers. The reduction can be carried out either in stereoselective way or not. The hydrogen donor can be formic acid and its derivatives, borane and its derivatives, cyclohexene and its derivatives or other hydrogen donor used in reduction of ketones. Reduction can also be carried out following biochemical principles. Next, **Vb** is transformed by first converting hydroxyl group to a better leaving group by either formation of substituted or unsubstituted arylsulfonate or substituted or unsubstituted alkylsulfonate ester and then to **VIb** by addition of a base, preferably potassium *tert*-butoxide or sodium hydride. Alternatively, the base used in the reaction from **Vb** to **VIb** can also be the same base as the one used for the transformation of hydroxyl group to a better leaving group as described above. In the last step, **VIb** is transformed to **VII** by hydrolysis in basic or acidic conditions, optionally in the presence of a cosolvent.

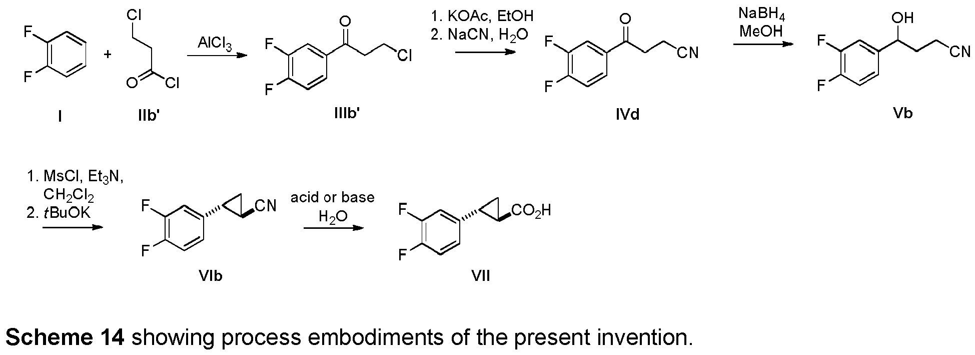

Alternatively, the compound with the activated hydroxyl group can optionally be isolated before base induced transformation to **VIb**.

Alternatively, the compound of formula **Vb** can be treated with phosphine derivatives such as trialkyl or triaryl phosphines, preferably triphenylphosphine and dialkyl diazodicarboxylate such as diethyl diazodicarboxylate (DEAD) in the presence of a base to yield the compound of formula **VIb**.

Further aspects of the present invention reside in the provision of valuable intermediate compounds of formula **V** and **VI**, and a salt of compound of formula **VII**, all useful in the synthesis of ticagrelor (**TCG**): 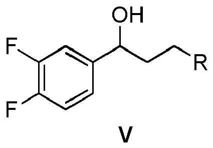 wherein R is a group convertible to carboxyl group selected from the group consisting of CN, trihalomethyl and COOR₁, wherein R₁ is linear or branched C₁-C₆ alkyl; 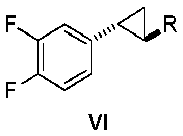 wherein R is a group convertible to carboxyl group selected from the group consisting of CN, trihalomethyl and COOR₁, wherein R₁ is linear or branched C₁-C₆ alkyl;

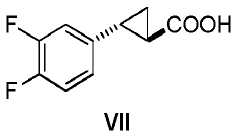

A suitable salt of intermediate **VII** is a metal salt, for example an alkali metal or earth alkali metal salt. The preferred salt of intermediate **VII** is lithium salt.

Particular examples of such useful intermediate compounds are listed by their respective formulas below:

| **Formula** | **Chemical name** |
|---|---|
| 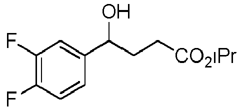 | isopropyl 4-(3,4-difluorophenyl)-4-hydroxybutanoate |
| 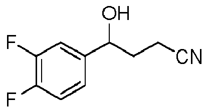 | 4-(3,4-difluorophenyl)-4-hydroxybutanenitrile |
| 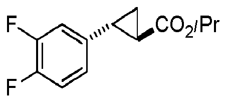 | isopropyl *trans*-2-(3,4-difluorophenyl)cyclopropanecarboxylate |
| 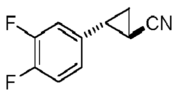 | *trans*-2-(3,4-difluorophenyl)cyclopropanecarbonitrile |
| 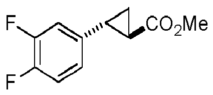 | methyl *trans*-2-(3,4-difluorophenyl)cyclopropanecarboxylate |
| 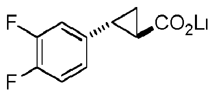 | lithium *trans*-2-(3,4-difluorophenyl)cyclopropanecarboxylate |

The following manner of presentation of structures of compounds VI and VII 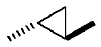 means that the position of substituents is *trans* and may contain a mixture of both enantiomers 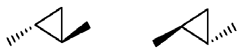 as a racemic mixture, a mixture enriched in one enantiomer or one particular enantiomer. The ratio of enantiomer mainly depends on enantioselectivity of reduction of the keto group in the compound of formula IV, which can be carried out by achiral reducing agents, such as borohydrides, by achiral agents such as boranes with chiral promotors such as with oxazaborolidine, by enantioselective reducing agents, by enantiomerically enriched chiral catalyst and hydrogen or hydrogen donor or by enzymatic way. Preferably the selection of reducing system should be carried out in such way that the synthesis leads to the intermediates with configuration at the position of the aryl substituent, which finally leads to cyclopropyl intermediates suitable for the synthesis of ticagrelor.

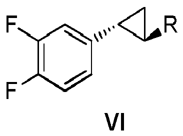

The enantiomerically enriched intermediates with suitable configuration at the position of the aryl substituent can be obtained also by other processes well known in the art, such as for example chromatography on chiral column, crystallization, crystallization by formation of diastereomeric salts or kinetic resolution.

In the following the present invention will be described in further detail by illustrative, nonlimiting examples.

### Examples

### Example 1: Preparation of 4-(3,4-difluorophenyl)-4-oxobutanoic acid (IIIa)

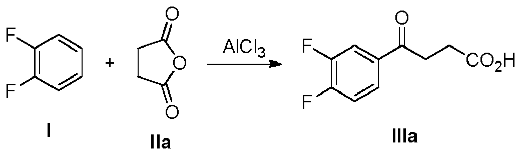

To a mixture of 1,2-difluorobenzene (I) (15 mL) and succinic anhydride (**IIa**) (4.0 g, 40.0 mmol) was slowly added AlCl₃ (16.0 g, 120 mmol). The resulting reaction mixture was stirred at 40°C for 16 h, then it was poured to the mixture of 37% HCl (20 mL) and ice (50 g). After extraction with MeTHF (3 x 20 mL), combined organic layers are dried over MgSO₄, and then concentrated to afford crude product, which was then recrystallized from toluene to give 6.6 g (77% yield) of title compound as white powder. Melting point 80°C; MS (ESI) m/z: 215 [MH]⁺.

### Example 2: Preparation of methyl 4-(3,4-difluorophenyl)-4-oxobutanoate (IVc)

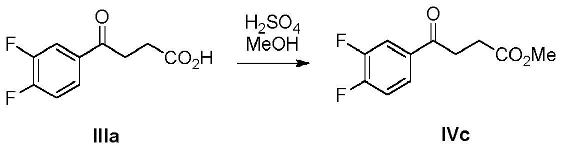

A mixture of **IIIa** (1.5 g, 7.0 mmol) and H₂SO₄ (96%, 0.15 g) in MeOH (20 mL) is stirred at 60°C for 2 hours, then the resulting reaction mixture was poured in the solution of K₂CO₃ (3 g) in water (50 mL). Ice (50 g) was added and mixture was stirred for 15 min. White solid was then filtered off, washed with water and dried to afford title compound as white powder (1.57 g, 98% yield). Melting point 46°C; MS (ESI) *m*/*z*: 229 [MH]⁺.

### Example 3: Preparation of isopropyl 4-(3,4-difluorophenyl)-4-oxobutanoate (IVa)

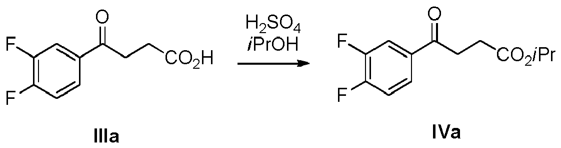

A mixture of **Ia** (1.5 g, 7.0 mmol) and H₂SO₄ (96%, 0.15 g) in iPrOH (20 mL) is stirred at 80°C for 16 hours, then the resulting reaction mixture was poured in the solution of K₂CO₃ (3 g) in water (50 mL). Ice (50 g) was added and mixture was stirred for 15 min. White solid was then filtered off, washed with water and dried to afford title compound as white powder (1.50 g, 84% yield). Melting point 36°C; MS (ESI) *m*/*z*: 257 [MH]⁺.

### Example 4: Preparation of methyl 4-(3,4-difluorophenyl)-4-hydroxybutanoate (Vc)

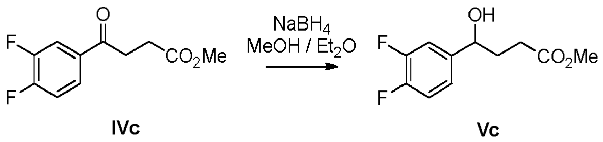

To a solution of **IVc** (1.0 g, 4.38 mmol) in MeOH (7 mL) / Et₂O (7 mL) NaBH₄ (83 mg, 2.19 mmol) was added at 0°C. The resulting reaction mixture was stirred at 0°C for 15 min, then saturated solution of NaHCO₃ (30 mL) was added, and product was extracted to THF (3 x 10 mL). Combined organic layers were dried over Na₂SO₄, and then concentrated. Purification by chromatography (SiO₂, hexane:EtOAc) afforded title compound as colorless oil (0.89 g, 88% yield). MS (ESI) *m*/*z*: 230 [MH]⁺.

### Example 5: Preparation of isopropyl 4-(3,4-difluorophenyl)-4-hydroxybutanoate (Va)

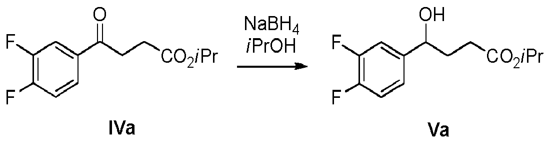

To a solution of **IVa** (1.4 g, 5.46 mmol) in iPrOH (20 mL) NaBH₄ (0.10 g, 2.73 mmol) was added at 0°C. The resulting reaction mixture is stirred at room temperature for 2 h, then AcOH (0,1 mL) and water (100 mL) were added, and product was extracted to THF (3 x 20 mL). Combined organic layers were dried over MgSO₄, and then concentrated. Purification by chromatography (SiO₂, hexane:EtOAc) afforded title compound as colorless oil (0.73 g, 56% yield). MS (ESI) *m*/*z*: 259 [MH]⁺.

### Example 6: Preparation of methyl trans-2-(3,4-difluorophenyl)cyclopropanecarboxylate (VIc)

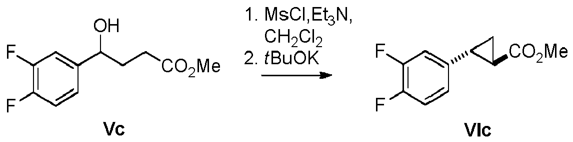

To a solution of **Vc** (0.89 g, 3.87 mmol) and triethylamine (1.08 mL, 7.74 mmol) in dry THF (15 mL) was added under stirring at 0°C MsCl (0.33 mL, 4.26 mmol). Resulting reaction mixture was stirred at 0°C for 20 min, then tBuOK (0.65 g, 5.81 mmol) was added, and reaction mixture was stirred at 0°C for additional 20 min. Then 1 M HCl (10 mL) was added, product was extracted to MeTHF (3 x 10 mL), combined organic phases were dried over MgSO₄, then concentrated to afford crude compound, which was then purified by chromatography (SiO₂, hexane:EtOAc) to afford title compound as colorless oil (0.52 g, 63%). MS (ESI) *m*/*z*: 213 [MH]⁺.

### Example 7: Preparation isopropyl trans-2-(3,4-difluorophenyl)cyclopropanecarboxylate (VIa)

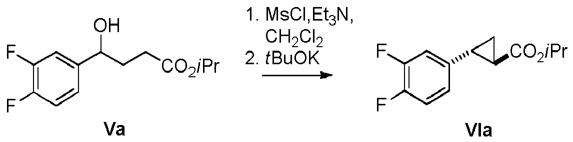

To a solution of **Va** (0.5 g, 1.94 mmol) and triethylamine (0.54 mL, 3.87 mmol) in dry THF (10 mL) was added under stirring at 0°C MsCl (0.17 mL, 2.13 mmol). Resulting reaction mixture was stirred at 0°C for 20 min, then tBuOK (0.43 g, 3.87 mmol) was added, and reaction mixture was stirred at 0°C for additional 20 min. Then 1 M HCl (10 mL) was added, product was extracted to MeTHF (3 x 10 mL), combined organic phases were dried over MgSO₄, then concentrated to afford crude compound, which was then purified by chromatography (SiO₂, hexane:EtOAc) to afford title compound as colorless oil (0.37 g, 79%). MS (ESI) *m*/*z*: 241 [MH]⁺.

### Example 8: Preparation of trans-2-(3,4-difluorophenyl)cyclopropanecarboxylic acid (VII)

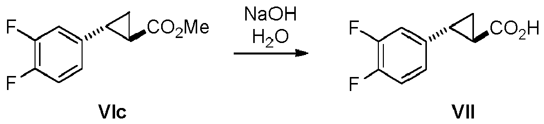

To a solution of **VIc** (0.36 g, 1.70 mmol) in EtOH (5 mL) was added 5 M NaOH (0.37 mL, 1.87 mmol). Resulting reaction mixture was stirred at room temperature for 16 h. 1 M HCl (20 mL) was added, product was extracted to MeTHF (3 x 10 mL), combined organic phases were dried over MgSO₄, and concentrated to afford white solid (0.28 g, 83% yield). MS (ESI) *m*/*z*: 199 [MH]⁺.

### Example 9: Preparation of trans-2-(3,4-difluorophenyl)cyclopropanecarboxylic acid (VII)

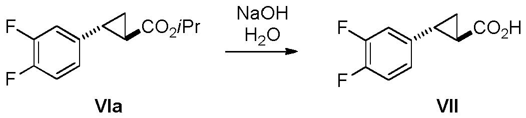

To a solution of **VIa** (100 mg, 0.42 mmol) in EtOH (5 mL) was added 8 M NaOH (0.1 mL). Resulting reaction mixture was stirred at room temperature for 16 h. 1 M HCl (5 mL) was added, product was extracted to MeTHF (3 x 5 mL), combined organic phases were dried over MgSO₄, and concentrated to afford white solid (61 mg, 73% yield).

### Example 10: Preparation of 3-chloro-1-(3,4-difluorophenyl)propan-1-one (IIIb')

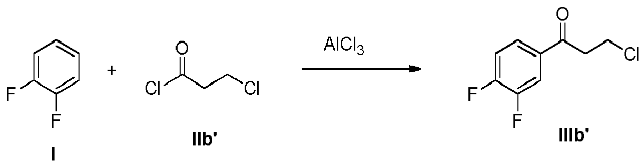

A mixture of AlCl₃ (160 g), 1,2-difluorobenzene (**I**) and 3-chloropropionyl chloride (**IIb'**) (96 mL) was stirred at 45 °C for 6 hours and the resulting mixture was poured into a mixture of ice (640 g) and concentrated HCl (60 mL). Another portion of ice (300 g) was added, the phases were separated and the organic phase was washed twice with dichloromethane (2 x 100 mL). Combined organic phases were washed twice with satureted aqueous NaHCO₃ (2 x 500 mL), dried with magnesium sulfate, filtered and concentrated under reduced pressure to give 206 g of 3-chloro-1-(3,4-difluorophenyl)propan-1-one (**IIIb'**) as an oil.

### Example 11: Preparation of 4-(3,4-difluorophenyl)-4-oxobutanenitrile (IVb)

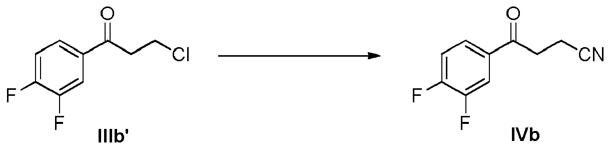

To a solution of 3-chloro-1-(3,4-difluorophenyl)propan-1-one (**IIIb'**) (20 g, 98 mmol) in ethyl acetate (50 mL) was added potassium tert-butoxide (9.60 g, 98 mmol) at 70 °C and stirred for 15 min and then cooled to 25 °C. The resulting mixture was filtered to remove salts (salts were washed with 5 mL of ethyl acetate and 10 mL of ethanol). To thus obtained was added dropwise a solution of sodium cyanide (9.6 g, 195 mmol) in water (50 mL) during 30 minutes. After the addition was complete the mixture was stirred at 25 °C for additional 0.5 hours, ethanol was added (20 mL) and the mixture was stirred for additional 3 hours. Phases were separated and the lower water layer was extracted with ethyl acetate (25 mL). The combined organic phases were washed with brine ( 3 × 25 mL) and concentrated under reduced pressure to give 18.5 g (97 %) 4-(3,4-difluorophenyl)-4-oxobutanenitrile (**IVb**) as oil that crystallized on standing. ¹H NMR (CDCl₃, 500 MHz): δ 2.78 (t, *J* = 7.1 Hz, 2H), 3.34 (t, *J* = 7.1 Hz, 2H), 7.29 (m, 1 H), 7.74 (m, 1H), 7.80 (m, 1H).

### Example 12: Preparation of 4-(3,4-difluorophenyl)-4-hydroxybutanenitrile (Vb)

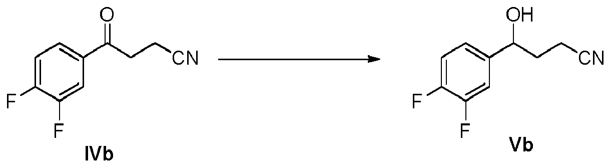

To a cold (0 °C) solution of 4-(3,4-difluorophenyl)-4-oxobutanenitrile (**IVb**) (10 g, 51 mmol) in methanol (100 mL) was added portion wise sodium borohydride (1.93 g) and the mixture was stirred at 5 °C for additional 30 minutes. Acetic acid (10 mL) was added drop wise and the methanol was removed under reduced pressure. Ethyl acetate (100 mL) was added and the resulting mixture was washed with water (3 x 30 mL), dried over magnesium sulfate, filtered and concentrated under reduced pressure. To the remaining oil was added methyl tert-butyl ether (30 mL, MTBE), filtered through the pad of silica gel (washed with some additional MTBE) and concentrated under reduced pressure. To the remaining oil was added toluene (20 mL) and again concentrated under reduced pressure to give 8.8 g (87 %) of 4-(3,4-difluorophenyl)-4-hydroxybutanenitrile (**Vb**) as an oil. ¹H NMR (CDCl₃, 500 MHz): δ 1.96 (m, 2H), 2.38 (m, 1H), 2.53 (m, 1 H), 4.75 (m, 1 H), 7.04 (m, 1H), 7.15 (m, 2H).

### Example 13: Preparation of trans-2-(3,4-difluorophenyl)cyclopropanecarbonitrile (VIb)

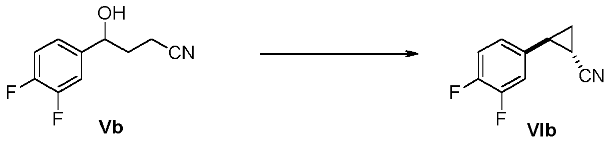

To a cold (0 °C) solution of 4-(3,4-difluorophenyl)-4-hydroxybutanenitrile (**Vb**) (5.7 g, 29 mmol) and triethylamine (8.0 mL, 57 mmol) in dry tetrahydrofurane (100 mL) was added methanesulfonyl chloride (2.5 mL, 32 mmol) and stirred at 0 °C for another 20 min. Potassium tert-butoxide (6.4 g, 57 mmol) was added and stirred at 0 °C for another 20 min. 1 M aqueous solution of HCl (57 mL) was added and the resulting solution was extracted with diethyl ether (3 x 70 mL). Combined ethereal layers were washed with brine (50 mL) and concentrated under reduced pressure. Crude product was purified by chromatography (silica gel; hexane/ethyl acetate 9 1 7 3) to give 2.84 g (55 %) of *trans*-2-(3,4-difluorophenyl)-cyclopropanecarbonitrile (**VIb**) as an oil. ¹H NMR (CDCl₃, 500 MHz): δ 1.41 (m, 1H), 1.53 (m, 1H), 1.64 (m, 1H), 2.60 (m, 1H), 6.87 (m, 1H), 6.92 (m, 1H), 7.10 (m, 1H).

### Example 14: Preparation of trans-2-(3,4-difluorophenyl)cyclopropanecarboxylic acid (VII)

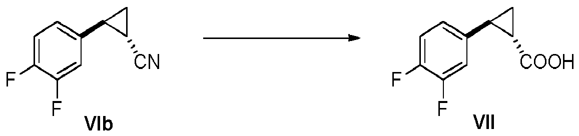

A mixture of trans-2-(3,4-difluorophenyl)cyclopropanecarbonitrile (**VIb**) (7.70 g) and 4 M aqueous lithium hydroxide (281 mL) was stirred at reflux temperature for 3.5 hours. The resulting mixture was cooled down and acidified with 4 M aqueous HCl to pH 1 and the product was extracted with MTBE (200 + 2 x 150 mL). Combined organic layers were dried with magnesium sulfate, filtered and concentrated under reduced pressure to give 8.12 g (95 %) of *trans*-2-(3,4-difluorophenyl)cyclopropanecarboxylic acid (**VII**) as a solid. ¹H NMR (CDCl₃, 500 MHz): δ 1.36 (m, 1H), 1.68 (m, 1H), 1.87 (m, 1H), 2.57 (m, 1H), 6.86 (m, 1H), 6.92 (m, 1H), 7.08 (m, 1 H).

### Example 15: Preparation of lithium trans-2-(3,4-difluorophenyl)cyclopropanecarboxylate (VIIa)

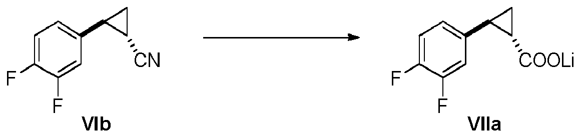

A mixture of trans-2-(3,4-difluorophenyl)cyclopropanecarbonitrile (**VIb**) (0.2 g) and 4 M aqueous lithium hydroxide (7.3 mL) was stirred at reflux temperature for 1.5 hours. The resulting mixture was cooled down, the solid was filtered off, washed with cold water to give 0.15 g (66 %) of lithium *trans*-2-(3,4-difluorophenyl)cyclopropanecarboxylate (**VIIa**) as a solid. ¹H NMR (DMSO-d6, 500 MHz): δ 0.89 (m, 1 H), 1.21 (m, 1 H), 1.53 (m, 1 H), 2.12 (m, 1 H), 6.89 (m, 1 H), 7.05 (m, 1 H), 7.24 (m, 1 H).

## Claims

1. A process for the preparation of a compound of formula **VI** 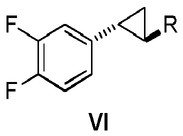 wherein R is an a group convertible to carboxyl group selected from the group consisting of CN, trihalomethyl and COOR₁, wherein R₁ is linear or branched C₁-C₆ alkyl,
the process comprising the steps of:
(i) providing a compound of formula **V** 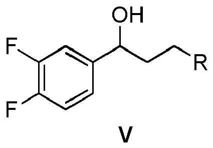 wherein R is a group convertible to carboxyl group selected from the group consisting of CN, trihalomethyl and COOR₁, wherein R₁ is linear or branched C₁-C₆ alkyl,
(ii) converting the hydroxyl group of the compound of formula **V** to a better leaving group, and
(iii) converting the obtained compound by cyclization in a presence of a base to provide a compound of formula **VI**.

2. The process according to claim 1, wherein R is selected from a group consisting of COOMe, COOiPr and CN.

3. The process according to claim 1, wherein the compound of formula **V** is prepared by comprising the steps of
(i) providing a compound of formula **IV** 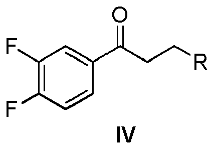 wherein R is as defined above, and (ii) reducing the keto group of the compound of formula **IV** to yield the compound of formula **V**.

4. The process according to claim 3, wherein the compound of formula **IV** is prepared by comprising the steps of:
(i) providing a compound of formula **I** 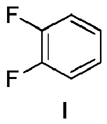
(ii) reacting the compound of formula I with a compound of formula **IIa** or **IIb** 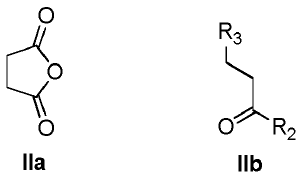 wherein R₂ and R₃ are independently selected from a group consisting of hydroxyl, Cl, Br and I,
to yield a compound of formula **IIIa** or **IIIb,** respectively 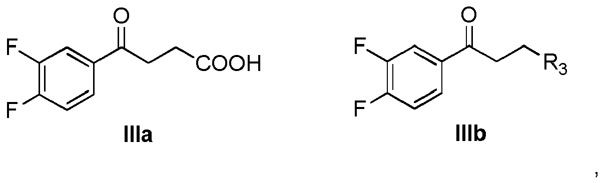 and
(iii) converting the compound of formula **IIIa** or **IIIb** to the compound of formula **IV**.

5. The process according to claim 1, wherein the compound of formula **VI** is further converted to a compound of formula **VII** or a salt thereof

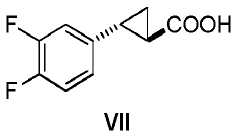

6. The process according to claim 5, wherein the compound of formula VII or a salt thereof is further converted to a compound of formula VIII or a salt thereof

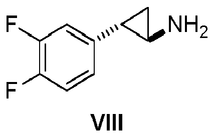

7. A compound of the following formula Va or Vb

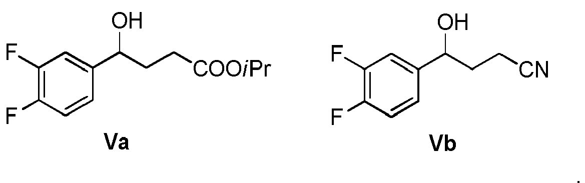

8. A compound of the following formula **VIa**, **VIb** or **VIc**

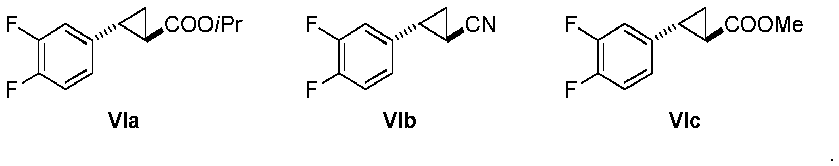

9. A metal salt of a compound of the following formula **VII**

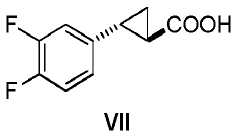

10. A compound of the following formula **VIIa**

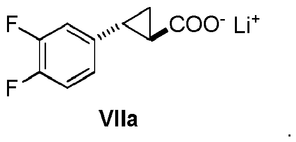

11. Use of a compound as defined in any one of claims 7 to 10, and their stereochemically isomeric forms, in the preparation of ticagrelor.
